# EUROPEAN PATENT APPLICATION

(11) **EP 1 968 131 A1**
(43) Date of publication of application: **10.09.2008**
(21) Application number: 06843089.1
(22) Date of filing: 22.12.2006
(51) Int. Cl.: H01L 51/50, C07D 487/04, C09K 11/06

(54) **MATERIAL FOR ORGANIC ELECTROLUMINESCENT DEVICE AND ORGANIC ELECTROLUMINESCENT DEVICE**

(30) Priority: 27.12.2005 JP 2005374629
(71) Applicant: Idemitsu Kosan Co., Ltd., Chiyoda-ku Tokyo 100-8321 (JP)
(72) Inventor: MORISHITA, Hironobu, Sodegaura-shi, Chiba 299-0293 (JP); HOSOKAWA, Chishio, Sodegaura-shi, Chiba 299-0293 (JP); KAWAMURA, Hisayuki, Sodegaura-shi, Chiba 299-0293 (JP)
(74) Representative: Gille Hrabal Struck Neidlein Prop Roos
(86) International application number: PCT/JP2006/325632
(87) International publication number: WO 2007/077766

(57) **Abstract**

A material for an organic electroluminescent device represented by the following formula (I): 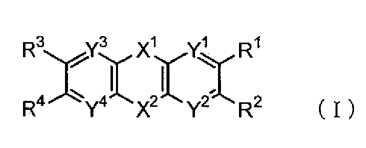 wherein X¹ and X² are independently one of Specific divalent groups; Y¹ to Y⁴ are independently a carbon atom or a nitrogen atom; R¹ to R⁴ are independently a hydrogen atom, an alkyl group, an aryl group, a heterocyclic group, a halogen atom, a fluoroalkyl group, or a cyano group; and R¹ and R², and R³ and R⁴ may be bonded together to form a ring, respectively.

## Description

### TECHNICAL FIELD

The invention relates to a material for an organic electroluminescent device and an organic electroluminescent device using the same.

### BACKGROUND ART

An organic electroluminescent device (hereinafter, "electroluminescent" is often abbreviated as "EL") is a self-emission device utilizing the principle that a fluorescent material emits light by the recombination energy of holes injected from an anode and electrons injected from a cathode when an electric field is applied.

Since C.W. Tang et al. of Eastman Kodak Co. reported a low-voltage driven organic EL device of stacked type (non-Patent Document 1, for example), studies on organic EL devices in which organic materials are used as constitution materials has been actively made.
The organic EL device reported by Tang et al. has a stacked structure in which tris(8-hydroxyquinolinol)aluminum is used as an emitting layer and a triphenyldiamine derivative is used as a hole-transporting layer. The advantage of the stacked structure include increased injection efficiency of holes to the emitting layer, increased generation efficiency of excitons generated by recombination while blocking electrons injected from the cathode, and containing the excitons generated in the emitting layer.

As the stacking structure of the organic EL device, a two-layer type formed of a hole-transporting (injecting) layer and an electron-transmitting layer, or a three-layer type formed of a hole-transporting (injecting) layer, an emitting layer, and an electron-transporting (injecting) layer or the like is well known. In such a device with a stacked structure, the device structures or the fabrication methods have been contrived to increase recombination efficiency of injected holes and electrons.

Heretofore, an aromatic diamine derivative as described in Patent Document 1 or an aromatic condensed ring diamine derivative as described in Patent Document 2 is known as a hole-transporting material used in an organic EL device.
However, to obtain a sufficient luminance in an organic EL device using the aromatic diamine derivative as the hole-transporting material, it is required to apply a high voltage. Applying a high voltage causes such problems as shortened lifetime of the device, increased power consumption, and the like.

To solve the problems, doping a hole-injection layer with an electron-accepting compound such as Lewis acid or the like has been proposed (Patent Documents 3 to 8, or the like). However, the electron-accepting compounds used in Patent Documents 3 to 6 have disadvantages that they are unstable to handle during fabricating an organic EL device, that the lifetime of an organic EL device fabricated using these compounds is shortened due to a lowering in stability such as heat resistance when an organic EL device is driven, and the like.
Tetrafluorodicyanoquinodimethane of an electron-accepting compound described in Patent Documents 5, and 7 to 8 is sublimed readily since it has a low molecular weight and is substituted with fluorine. Therefore, tetrafluorodicyanoquinodimethane may diffuse within an apparatus when fabricating an organic EL device by vacuum deposition, causing the apparatus or the device to be contaminated.

Patent Document 1: U.S.P. No. 4,720,432
Patent Document 2: U.S.P. No. 5,061,569
Patent Document 3: JP-A-2003-031365
Patent Document 4: JP-A-2001-297883
Patent Document 5: JP-A-2000-196140
Patent Document 6: JP-A-11-251067
Patent Document 7: JP-A-4-297076
Patent Document 8: JP-T-2004-514257
Non-patent document 1: Applied Physics Letters, 51, 913 (1987)

### DISCLOSURE OF THE INVENTION

The invention has been made based on the above problems. An object of the invention is to provide an electron-receiving material suitable as a constitution material of an organic EL device.

The inventors made extensive studies, and noticed compounds having a thioxanthene skeleton or a pyrazino skeleton. Since these compounds have a skeleton similar to anthraquinone, with a resonance structure spreading within the ring, anion radicals of these compounds are found to be stable as a result of an ESR measurement or an electrochemical measurement (Z. Naturforsch, Vol. 46b, p. 326 to 338, J. Am. Chem. Soc., Vol. 85, p. 1821, or the like). In addition, since these compounds are excellent in heat resistance, it is expected that deposition stability when fabricating an organic EL device can be increased or heat deterioration when driving an organic EL device can be suppressed.
As a result of further extensive studies, the inventors have found that a specific compound of the above compounds can be an electron-accepting material suited to an organic EL device. The inventors have also found that an organic EL device using the above compounds can be driven at a low voltage and can exhibit a long lifetime.

The invention provides the following material for an organic EL device, and the like.
1. A material for an organic electroluminescent device represented by the following formula (I): 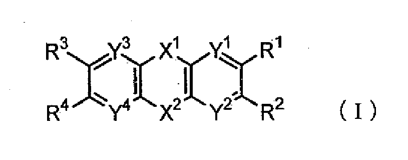 wherein X¹ and X² are independently one of the following divalent groups; Y¹ to Y⁴ are independently a carbon atom or a nitrogen atom; R¹ to R⁴ are independently a hydrogen atom, an alkyl group, an aryl group, a heterocyclic group, a halogen atom, a fluoroalkyl group, or a cyano group; and R¹ and R², and R³ and R⁴ may be bonded together to form a ring, respectively. 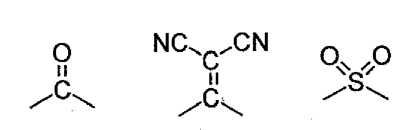
2. The material for an organic electroluminescent device according to 1, which is a compound represented by the following formula (II) or (III): 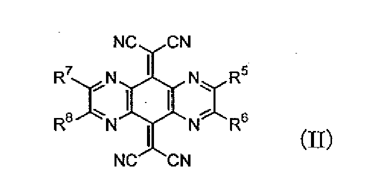 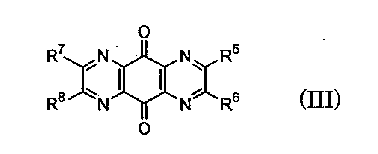 wherein R⁵ to R⁸ are independently a hydrogen atom, an alkyl group, an aryl group, a heterocyclic group, a halogen atom, a fluoroalkyl group or a cyano group; and R⁵ and R⁶, and R⁷ and R⁸ may be bonded together to form a ring, respectively.
3. The material for an organic electroluminescent device according to 1, which is a compound represented by the following formula (IV): 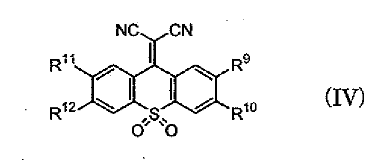 wherein R⁹ to R¹² are independently a hydrogen atom, an aryl group, a heterocyclic group, a halogen atom, a fluoroalkyl group, or a cyano group.
4. The material for an organic electroluminescent device according to any one of 1 to 3, wherein an acetonitrile solution has a reduction potential of -0.5 V (vsSCE) or more.
5. An organic electroluminescent device comprising a cathode and an anode, and one or a plurality of organic thin layers including an emitting layer between the cathode and the anode;
   at least one of the organic thin layers containing the material for an organic electroluminescent device according to any one of 1 to 4.
6. The organic electroluminescent device according to 5, wherein the organic thin layers are a multilayer body in which a hole-transporting layer, an emitting layer, and an electron-transporting layer are stacked in this order from the anode.
7. The organic electroluminescent device according to 6, wherein the hole-transporting layer contains the material for an organic electroluminescent device according to any one of 1 to 4.
8. The organic electroluminescent device according to 5, wherein the organic thin layers are a multilayer body in which a hole-injection layer, a hole-transporting layer, an emitting layer, and an electron-transporting layer are stacked in this order from the anode, and the hole-injection layer contains the material for an organic electroluminescent device according to any one of 1 to 4.
9. The organic electroluminescent device according to 7 or 8, wherein the hole-transporting layer or the hole-injecting layer containing the material for an organic electroluminescent device further contains a phenylenediamine compound represented by the following formula (V): 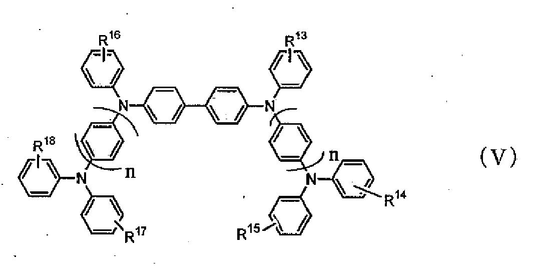
wherein R¹³ to R¹⁸ are independently a hydrogen atom, a halogen atom, a trifluoromethyl group, an alkyl group, an aryl group, or a heterocyclic group or R¹³ to R¹⁸ may form a naphthalene skeleton, a carbazole skeleton, or a fluorene skeleton with a phenyl group bonded; and n represents 1 or 2.

According to the invention, a novel material for an organic EL device is provided. Also, according to the invention, an organic EL device which can be driven at a low voltage and has a long lifetime is provided.

### BRIEF DESCRIPTION OF THE DRAWING

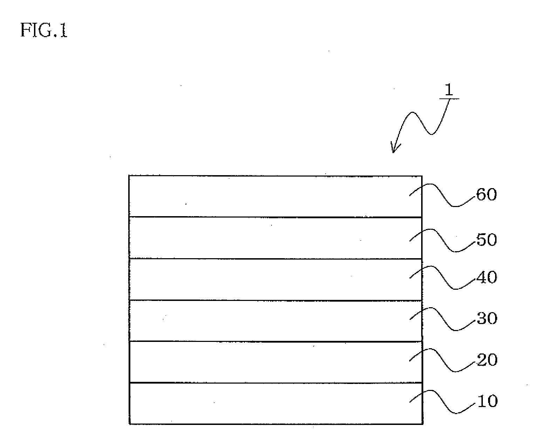

FIG. 1 is a systematic cross-sectional view showing one embodiment of the organic EL device of the invention.

### BEST MODE FOR CARRYING OUT THE INVENTION

Firstly, the material for an organic EL device of the invention will be described.
The material for an organic EL device is a compound represented by the following formula (I): 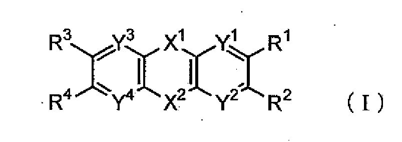 In the formula (I), X¹ and X² are independently one of divalent groups shown below:

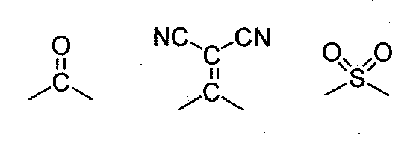

Y¹ to Y⁴ are independently a carbon atom or a nitrogen atom, and R¹ to R⁴ are independently a hydrogen atom, an alkyl group, an aryl group, a heterocyclic group, a halogen atom, a fluoroalkyl group, or a cyano group.

The compound represented by the formula (I) has electron-receiving properties. Therefore, when using in an organic EL device, the organic EL device can be driven at a low voltage and can exhibit a prolonged lifetime.
In addition, since the compound does not scatter within a film-forming apparatus during manufacturing an organic EL device, the film-forming apparatus or the organic EL device is not contaminated.

As examples of the alkyl group represented by R¹ to R⁴, methyl, ethyl, propyl, tert-butyl, cyclohexyl, adamantyl, and the like can be given. Of these, methyl, tert-butyl, and cyclohexyl are preferable.

As examples of the aryl group represented by R¹ to R⁴, phenyl, naphthyl, tolyl, 4-fluorophenyl, 4-fluoromethylphenyl, 4-cyano-phenyl, fluorene, and the like can be given.
Of these, phenyl, 4-fluorophenyl, and 4-trifluoromethyl-phenyl are preferable.

As examples of the heterocyclic group represented by R¹ to R⁴, pyridine, pyrimidine, imidazole, quinoline, imidazopyridine, and the like can be given. Of these, pyridine is preferable.

As examples of the halogen atom represented by R¹ to R⁴, fluorine and chlorine are preferable.

As examples of the fluoroalkyl group represented by R¹ to R⁴, trifluoromethyl, pentafluoroethyl, perfluorohexyl, perfluoroadamantyl, and the like can be given. Of these, trifluoromethyl is preferable.

R¹ and R², and R³ and R⁴ may be bonded together to form a ring, respectively. For example, rings shown below are formed. Of these, cyclohexane is preferable.

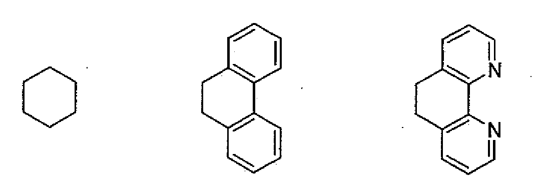

Of the compounds represented by the formula (I), compounds represented by the following formula (II) or (III) are preferable. 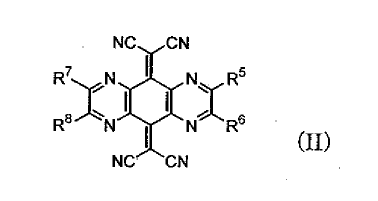 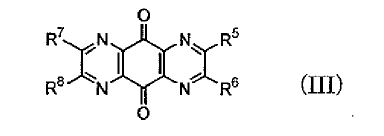 wherein R⁵ to R⁸ are independently hydrogen, alkyl, aryl, heterocyclic, halogen, fluoroalkyl, or cyano. R⁵ and R⁶, and R⁷ and R⁸ may be bonded together to form a ring, respectively.
Specific examples of the alkyl, aryl, heterocyclic, halogen, and fluoroalkyl represented by R⁵ to R⁸ are the same as those for R¹ to R⁴ of the formula (I) as described above. Specific examples of the ring formed by bonding of R⁵ and R⁶, and R⁷ and R⁸ are the same as those formed by bonding of R¹ and R², and R³ and R⁴.

As the electron-accepting compound represented by the formula (I), a compound represented by the following formula (IV) can be suitably used. 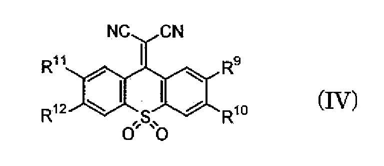 wherein R⁹ to R¹² are independently hydrogen, alkyl, aryl, heterocyclic, halogen, fluoroalkyl, or cyano. Specific examples of the alkyl, aryl, heterocyclic, halogen, and fluoroalkyl represented by R⁹ to R¹² are the same as those for R¹ to R⁴ of the formula (I) as described above.

The material for an organic EL device of the invention has a reduction potential in an acetonitrile solution of preferably -0.5V (vsSCE) or more.
The electron-receiving properties can be further increased by using a compound with a reduction potential of -0.5V or more.
Preferred examples of the organic EL device of the invention are given below. Examples of a method of synthesizing the material for an organic El device will be described in detail in the examples given later.

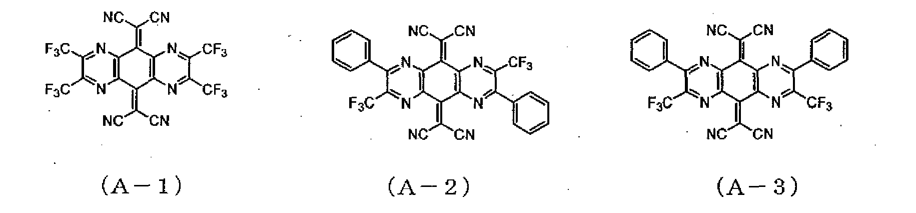

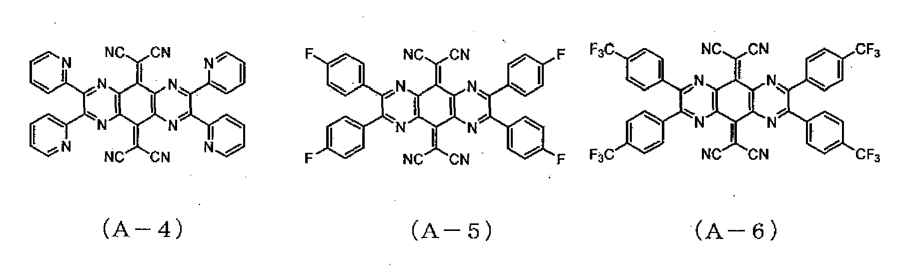

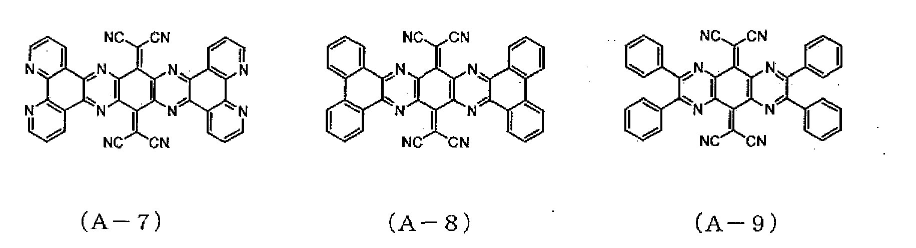

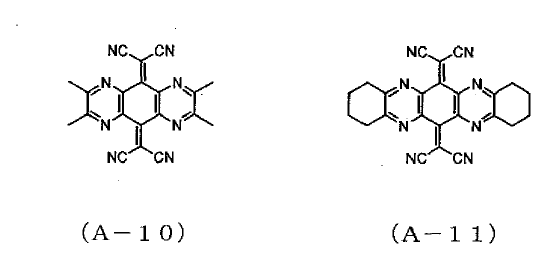

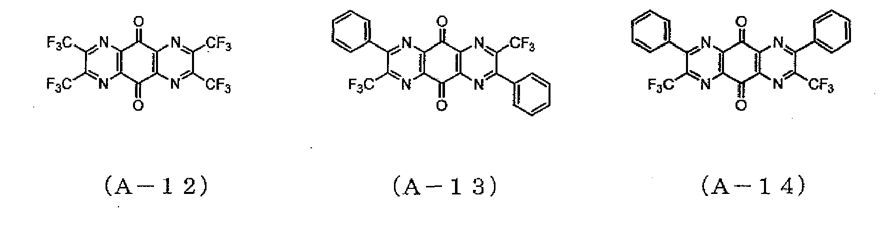

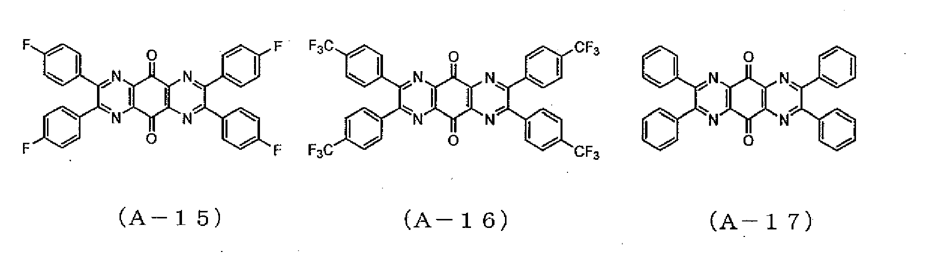

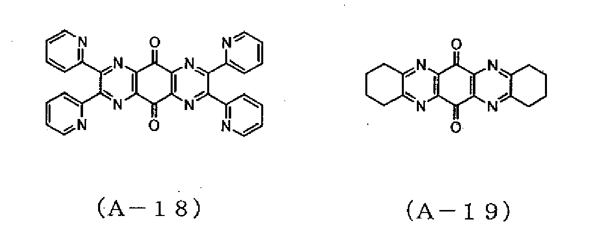

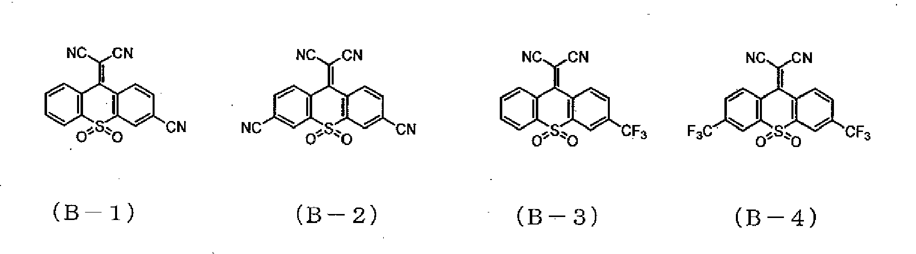

Next, the organic EL device of the invention will be described.
The organic EL device of the invention has one or a plurality of organic thin layers including an emitting layer being interposed between a cathode and an anode. At least one layer constituting the organic thin layers contains the material for an organic EL device of the invention.

FIG. 1 is a systematic cross-sectional view showing one embodiment of the organic EL device of the invention.
In the organic EL device 1, an anode 10, a hole-injecting layer 20, a hole-transporting layer 30, an emitting layer 40, an electron-transporting layer 50, and a cathode 60 are stacked on a substrate (not shown) in this order. In this device, the organic thin layer has a stacked structure of the hole-injecting layer 20, the hole-transporting layer 30, the emitting layer 40, and the electron-transporting layer 50. At least one of the layers constituting the organic thin layers contains the material for an organic EL device of the invention. This structure leads to a lowered driving voltage and a prolonged lifetime of an organic EL device.
The content of the material for an organic EL device in the layer constituting organic thin layers containing the material for an organic EL device of the invention is preferably 1 to 100 mol%.

In the organic EL device of the invention, it is preferred that a layer which is present in a region (hole-transporting region) between the anode 10 and the emitting layer 40, specifically, the hole-injection layer 20 or the hole-transporting layer 30, contain the material for an organic EL device of the invention. In the device having both the hole-injecting layer 20 and the hole-transporting layer 30 like the embodiment, it is preferred that the hole-injection layer 20 nearer the anode contain the material for an organic EL device of the invention.

When the material for an organic EL device of the invention is used in the layer present in the hole-transporting region, the material for an organic EL device of the invention may form the hole-injecting layer or the hole-transporting layer singly or in combination with other materials.
For example, when the material for an organic EL device of the invention and an aromatic amine derivative are mixed to form the hole-injection layer or the hole-transporting layer, it is preferable to use a phenylenediamine compound represented by the formula (V). 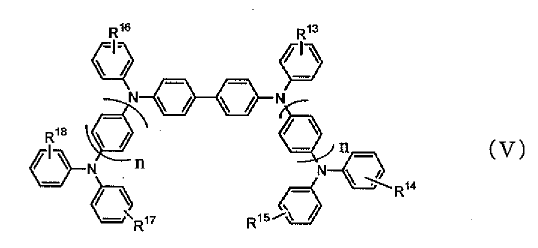 wherein R¹³ to R¹⁸ are independently a hydrogen atom, a halogen atom, a trifluoromethyl group, an alkyl group, an aryl group, or a heterocyclic group, or R¹³ and R¹⁸ form a naphthalene skeleton, a carbazole skeleton, or a fluorene skeleton with a phenyl group bonded; and n represents 1 or 2.
If the above phenylenediamine compound is contained in combination, uniformity, heat resistance, or carrier-injection properties of the film may be improved as compared with a case where the material for an organic EL device of the invention is contained singly.

In the formula (V), fluorine is preferable as the halogen atom represented by R¹³ to R¹⁸.

As the alkyl group represented by R¹³ to R¹⁸, methyl, isopropyl, tert-butyl, and cyclohexyl are preferred, for example.

As the aryl group represented by R¹³ to R¹⁸, phenyl, naphthyl, and fluorenyl are preferable, for example. These groups may be substituted with methyl or the like.

As the heterocyclic group represented by R¹³ to R¹⁸, pyridine and pyrazine are preferable, for example.

R¹³ to R¹⁸ may form a naphthalene skeleton, a carbazole skeleton, or a fluorene skeleton with a phenyl group bonded. These skeletons may be substituted with methyl or the like.

The content of the compound represented by the formula (V) in the hole-transporting layer or the hole-injecting layer is preferably 0.1 to 98 mol%.
Preferred examples of the compound (V) are given below.

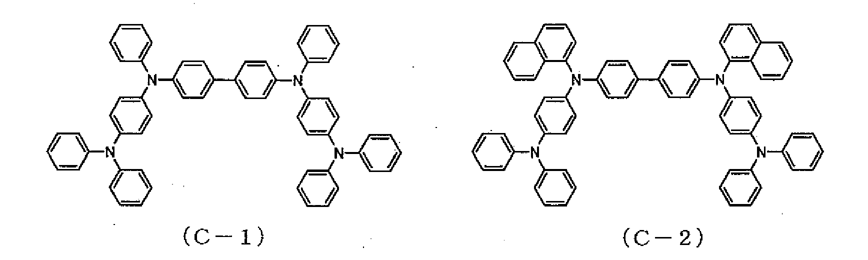

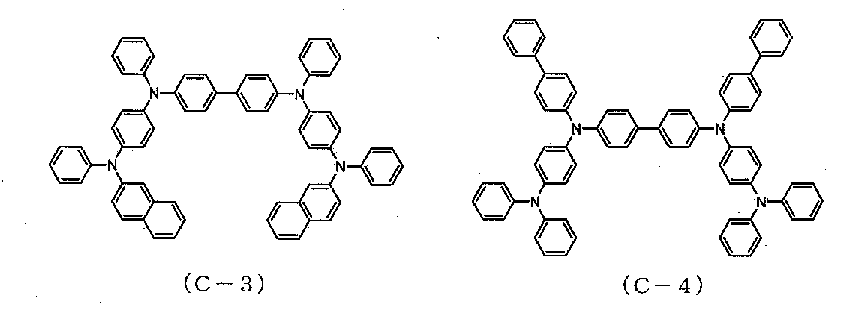

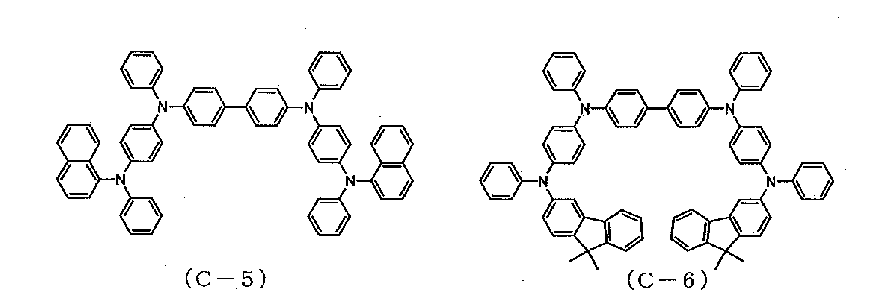

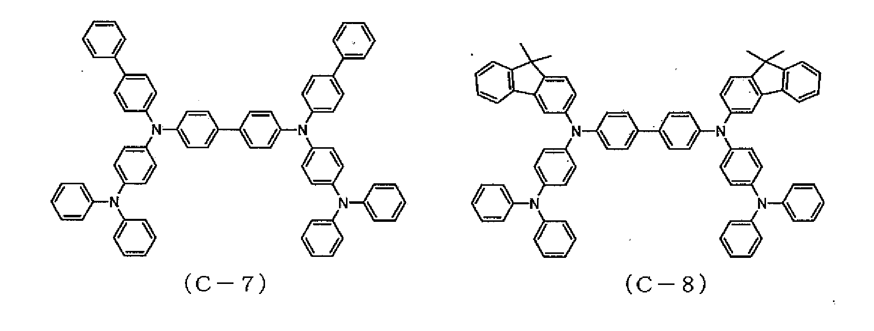

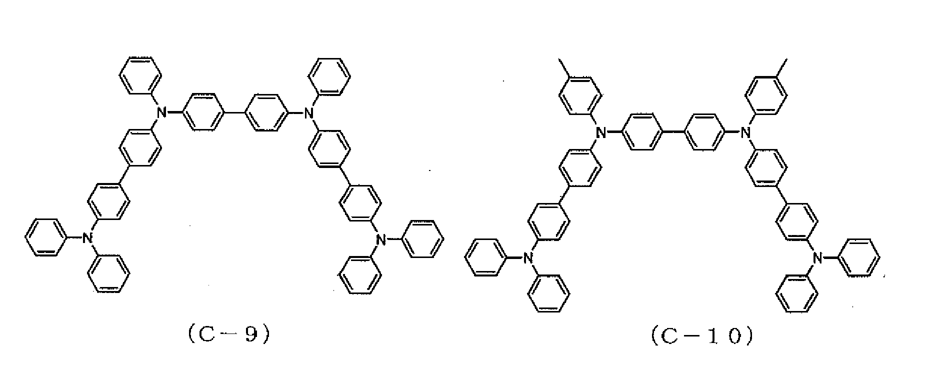

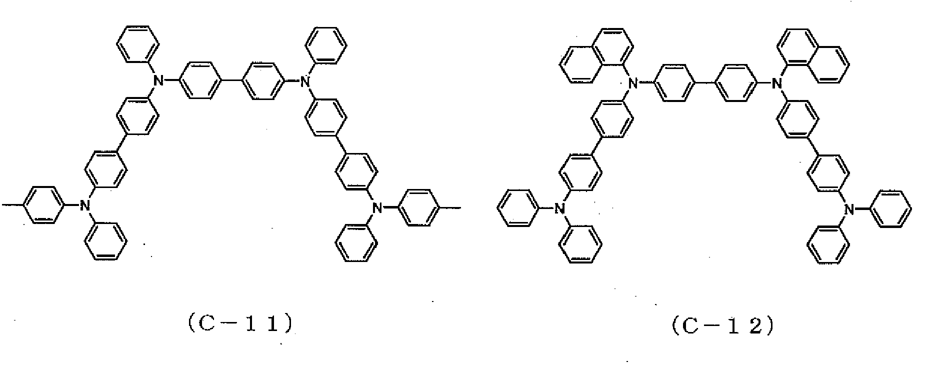

The structure of the organic EL device of the invention is not limited to the embodiment 1 described above. For example, the organic EL device of the invention may have structures (1) to (15) shown below.
(1) Anode/emitting layer/cathode
(2) Anode/emitting layer/cathode
(3) Anode/hole-transporting layer/emitting layer/electron-injecting layer/cathode
(4) Anode/hole-transporting layer/emitting layer/electron-transporting layer/cathode
(5) Anode/hole-transporting layer/adhesion-improving layer/cathode
(6) Anode/hole-injecting layer/hole-transporting layer/emitting layer/electron-transporting layer/cathode (FIG. 1)
(7) Anode/hole-transporting layer/emitting layer/electron-transporting layer/electron-injecting layer/cathode
(8) Anode/hole-injecting layer/hole-transporting layer/emitting layer/electron-transporting layer/electron-injecting layer/cathode
(9) Anode/insulative layer/hole-transporting layer/emitting layer/electron-transporting layer/cathode
(10) Anode/hole-transporting layer/emitting layer/electron-transporting layer/insulative layer/cathode
(11) Anode/inorganic semiconductor layer/insulative layer/hole-transporting layer/emitting layer/insulative layer/cathode
(12) Anode/insulative layer/hole-transporting layer/emitting layer/electron-transporting layer/insulative layer/cathode
(13) Anode/hole-injecting layer/hole-transporting layer/emitting layer/electron-transporting layer/insulative layer/cathode
(14) Anode/insulative layer/hole-injecting layer/hole-transporting layer/emitting layer/electron-transporting layer/electron-injecting layer/cathode
(15) Anode/insulative layer/hole-injecting layer/hole-transporting layer/emitting layer/electron-transporting layer/electron-injecting layer/insulative layer/cathode

Of these, the structures (4), (6), (7), (8), (12), (13), and (15) are preferably used.
Each member constituting the organic EL device of the invention will be described below.

### [Transparent substrate]

The organic EL device of the invention is formed on a transparent substrate. The transparent substrate is a substrate for supporting the organic EL device, and is preferably a flat and smooth substrate having a transmittance of 50% or more to light rays within visible ranges of 400 to 700 nm.
Specific examples thereof include glass plates and polymer plates. Examples of the glass plate include soda-lime glass, barium/strontium-containing glass, lead glass, aluminosilicate glass, borosilicate glass, barium borosilicate glass, and quartz. Examples of the polymer plate include polycarbonate, acrylic polymer, polyethylene terephthalate, polyethersulfide, and polysulfone.
Transparency is not required when the supporting substrate is positioned in the opposite direction to the light-outcoupling direction.

### [Anode]

The anode of the organic thin film EL device plays a role for injecting holes into its hole-transporting layer or emitting layer. When transparency is required for the anode, indium tin oxide alloy (ITO), tin oxide (NESA), zinc tin oxide alloy (IZO), gold, silver, platinum, copper, and the like may be used as the material for the anode. When a reflective electrode which does not require transparency is used, a metal such as aluminum, molybdenum, chromium, and nickel or alloys thereof may also be used.
Although these materials may be used individually, alloys thereof or materials wherein another element is added to the materials can be appropriately selected for use.
The anode can be formed by forming these electrode materials into a thin film by vapor deposition, sputtering or the like.
In the case where emission from the emitting layer is outcoupled through the anode, the transmittance of the anode with respect to the emission is preferably more than 10%. The sheet resistance of the anode is preferably several hundred Ω/□ or less. The film thickness of the anode, which is varied depending upon the material thereof, is usually from 10 nm to 1 µm, preferably from 10 to 200 nm.

### [Emitting layer]

The emitting layer of the organic EL device has the following functions in combination.
(1) Injecting function: function of allowing injection of holes from anode or hole injecting/transporting layer and injection of electrons from cathode or electron injecting/transporting layer upon application of electric field
(2) Transporting function: function of moving injected carriers (electrons and holes) due to force of electric field
(3) Emitting function: function of providing a site for recombination of electrons and holes to emit light

Note that electrons and holes may be injected into the emitting layer with different degrees, or the transportation capabilities indicated by the mobility of holes and electrons may differ. It is preferable that the emitting layer move either electrons or holes.

As the method of forming the emitting layer, a known method such as deposition, spin coating, or an LB method may be applied. It is preferable that the emitting layer be a molecular deposition film.
The term "molecular deposition film" refers to a thin film formed by depositing a vapor-phase material compound or a film formed by solidifying a solution-state or liquid-phase material compound. The molecular deposition film is distinguished from a thin film (molecular accumulation film) formed using the LB method by the difference in aggregation structure or higher order structure or the difference in function due to the difference in structure.
The emitting layer may also be formed by dissolving a binder such as a resin and a material compound in a solvent to obtain a solution, and forming a thin film of the solution by spin coating or the like, as disclosed in JP-A-57-51781.

As materials for the emitting layer, known emitting materials having a long life may be used. It is preferable to use a material represented by the formula (VI) as the emitting material. 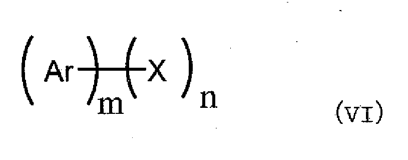 wherein Ar is an aromatic ring having 6 to 50 nucleus carbon atoms or a heteroaromatic ring having 5 to 50 nucleus atoms; X is a substituent; m is an integer of 1 to 5; and n is an integer of 0 to 6.

As specific examples of an aromatic ring and a heteroaromatic ring represented by Ar, a phenyl ring, a naphthyl ring, an anthracene ring, a biphenylene ring, an azulene ring, an acenaphthylene ring, a fluorene ring, a phenanthrene ring, a fluoranthene ring, an aceanthrylene ring, a triphenylene ring, a pyrene ring, a chrysene ring, a benzanthracene ring, a naphthacene ring, a picene ring, a perylene ring, a pentaphene ring, a pentacene ring, a tetraphenylene ring, a hexaphene ring, a hexacene ring, a rubicene ring, a coronene ring, a trinaphthylene ring, a pyrrole ring, an indole ring, a carbazole ring, an imidazole ring, a benzimidazole ring, an oxadizole ring, a triazole ring, a pyridine ring, a quinoxaline ring, a quinoline ring, a pyrimidine ring, a triazine ring, a thiophene ring, a benzothiophene ring, a thianthrene ring, a furan ring, a benzofuran ring, a pyrazole ring, a pyrazine ring, a pyridazine ring, an indolizine ring, a quinazoline ring, a phenanthroline ring, a silole ring, a benzosilole ring, and the like can be given.

Ar is preferably a phenyl ring, a naphthyl ring, an anthracene ring, an acenaphthylene ring, a fluorene ring, a phenanthrene ring, a fluoranthene ring, a triphenylene ring, a pyrene ring, a chrysene ring, a benzanthracene ring, or a perylene ring.

Specific examples of the substituents represented by X include a substituted or unsubstituted aromatic group having 6 to 50 nucleus carbon atoms, a substituted or unsubstituted aromatic heterocyclic group having 5 to 50 nucleus atoms, a substituted or unsubstituted alkyl group having 1 to 50 carbon atoms, a substituted or unsubstituted alkoxy group having 1 to 50 carbon atoms, a substituted or unsubstituted aralkyl group having 1 to 50 carbon atoms, a substituted or unsubstituted aryloxy group having 5 to 50 nucleus atoms, a substituted or unsubstituted arylthio group having 5 to 50 nucleus atoms, a substituted or unsubstituted carboxyl group having 1 to 50 carbon atoms, a substituted or unsubstituted styryl group, a halogen group, a cyano group, a nitro group, a hydroxyl group, or the like.

As examples of the substituted or unsubstituted aromatic group having 6 to 50 nucleus atoms, a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4" -t-butyl-p-terphenyl-4-yl group, 2-fluorenyl group, 9,9-dimethyl-2-fluorenyl group, 3-fluoranthenyl group, and the like can be given.

The substituted or unsubstituted aromatic group having 6 to 50 nucleus atoms is preferably a phenyl group, 1-naphthyl group, 2-naphthyl group, 9-phenanthryl group, 1-naphthacenyl group, 2-naphthacenyl group, 9-naphthacenyl group, 1-pyrenyl group, 2-pyrenyl group, 4-pyrenyl group, 2-biphenylyl group, 3-biphenylyl group, 4-biphenylyl group, o-tolyl group, m-tolyl group, p-tolyl group, p-t-butylphenyl group, 2-fluorenyl group, 9,9-dimethyl-2-fluorenyl group, 3-fluoranthenyl group, or the like.

As examples of the substituted or unsubstituted aromatic heterocyclic group having 5 to 50 nucleus atoms, a 1-pyrrolyl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 2-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 9-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-y1 group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthxolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 10-phenothiadinyl group, 1-phenoxadinyl group, 2-phenoxadinyl group, 3-phenoxadinyl group, 4-phenoxadinyl group, 10-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butyl-pyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, 4-t-butyl-3-indolyl group, and the like can be given.

Examples of the substituted or unsubstituted alkyl groups having 1 to 50 nucleus atoms include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, 1,2,3-trinitropropylcyclopropyl, cyclobutyl, cyclopentyl, cyclohexyl, 4-methylcyclohexyl, 1-adamanthyl, 2-adamanthyl, 1-norbornyl, and 2-norbornyl.

The substituted or unsubstituted alkoxy groups having 1 to 50 carbon atoms are groups represented by -OY. Examples of Y include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, and 1,2,3-trinitropropyl groups.

Examples of the substituted or unsubstitued aralkyl groups having 1 to 50 carbon atoms include benzyl, 1-phenylethyl, 2-phenylethyl, 1-phenylisopropyl, 2-phenylisopropyl, phenyl-t-butyl, α-naphthylmethyl, 1-α-naphthylethyl, 2-α-naphthylethyl, 1-α-naphthylisopropyl, 2-α-naphthylisopropyl, β-naphthylmethyl, 1-β-naphthylethyl, 2-β-naphthylethyl, 1-β-naphthylisopropyl, 2-β-naphthylisopropyl, 1-pyrrolylmethyl, 2-(1-pyrrolyl)ethyl, p-methylbenzyl, m-methylbenzyl, o-methylbenzyl, p-chlorobenzyl, m-chlorobenzyl, o-chlorobenzyl, p-bromobenzyl, m-bromobenzyl, o-bromobenzyl, p-iodobenzyl, m-iodobenzyl, o-iodobenzyl, p-hydroxybenzyl, m-hydroxybenzyl, o-hydroxybenzyl, p-aminobenzyl, m-aminobenzyl, o-aminobenzyl, p-nitrobenzyl, m-nitrobenzyl, o-nitrobenzyl, p-cyanobenzyl, m-cyanobenzyl, o-cyanobenzyl, 1-hydroxy-2-phenylisopropyl, and 1-chloro-2-phenylisopropyl groups.

The substituted or unsubstituted aryloxy group having 5 to 50 nucleus atoms is represented by -OY'. Examples of Y' include a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, and 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4''-t-butyl-p-terphenyl-4-yl group, 2-pyrrolyl group, 3-pyrrolyl group, pyrazinyl group, 2-pyridinyl group, 3-pyridinyl group, 4-pyridinyl group, 1-indolyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group; 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 1-phenoxadinyl group, 2-phenoxadinyl group, 3-phenoxadinyl group, 4-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butyl-pyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, and 4-t-butyl-3-indolyl group.

The substituted or unsubstituted arylthio group having 5 to 50 nucleus atoms is represented by -SY''. Examples of Y'' include a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, 9-phenanthryl group, 1-naphthacenyl group, 2-naphthacenyl group, 9-naphthacenyl group, and 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-pyrrolyl group, 3-pyrrolyl group, pyrazinyl group, 2-pyridinyl group, 3-pyridinyl group, 4-pyridinyl group, 2-indolyl group, 3-indolyl group, 4-indolyl group, 5-indolyl group, 6-indolyl group, 7-indolyl group, 1-isoindolyl group, 3-isoindolyl group, 4-isoindolyl group, 5-isoindolyl group, 6-isoindolyl group, 7-isoindolyl group, 2-furyl group, 3-furyl group, 2-benzofuranyl group, 3-benzofuranyl group, 4-benzofuranyl group, 5-benzofuranyl group, 6-benzofuranyl group, 7-benzofuranyl group, 1-isobenzofuranyl group, 3-isobenzofuranyl group, 4-isobenzofuranyl group, 5-isobenzofuranyl group, 6-isobenzofuranyl group, 7-isobenzofuranyl group, 2-quinolyl group, 3-quinolyl group, 4-quinolyl group, 5-quinolyl group, 6-quinolyl group, 7-quinolyl group, 8-quinolyl group, 1-isoquinolyl group, 3-isoquinolyl group, 4-isoquinolyl group, 5-isoquinolyl group, 6-isoquinolyl group, 7-isoquinolyl group, 8-isoquinolyl group, 2-quinoxalinyl group, 5-quinoxalinyl group, 6-quinoxalinyl group, 1-carbazolyl group, 2-carbazolyl group, 3-carbazolyl group, 4-carbazolyl group, 1-phenanthridinyl group, 2-phenanthridinyl group, 3-phenanthridinyl group, 4-phenanthridinyl group, 6-phenanthridinyl group, 7-phenanthridinyl group, 8-phenanthridinyl group, 9-phenanthridinyl group, 10-phenanthridinyl group, 1-acridinyl group, 2-acridinyl group, 3-acridinyl group, 4-acridinyl group, 9-acridinyl group, 1,7-phenanthrolin-2-yl group, 1,7-phenanthrolin-3-yl group, 1,7-phenanthrolin-4-yl group, 1,7-phenanthrolin-5-yl group, 1,7-phenanthrolin-6-yl group, 1,7-phenanthrolin-8-yl group, 1,7-phenanthrolin-9-yl group, 1,7-phenanthrolin-10-yl group, 1,8-phenanthrolin-2-yl group, 1,8-phenanthrolin-3-yl group, 1,8-phenanthrolin-4-yl group, 1,8-phenanthrolin-5-yl group, 1,8-phenanthrolin-6-yl group, 1,8-phenanthrolin-7-yl group, 1,8-phenanthrolin-9-yl group, 1,8-phenanthrolin-10-yl group, 1,9-phenanthrolin-2-yl group, 1,9-phenanthrolin-3-yl group, 1,9-phenanthrolin-4-yl group, 1,9-phenanthrolin-5-yl group, 1,9-phenanthrolin-6-yl group, 1,9-phenanthrolin-7-yl group, 1,9-phenanthrolin-8-yl group, 1,9-phenanthrolin-10-yl group, 1,10-phenanthrolin-2-yl group, 1,10-phenanthrolin-3-yl group, 1,10-phenanthrolin-4-yl group, 1,10-phenanthrolin-5-yl group, 2,9-phenanthrolin-1-yl group, 2,9-phenanthrolin-3-yl group, 2,9-phenanthrolin-4-yl group, 2,9-phenanthrolin-5-yl group, 2,9-phenanthrolin-6-yl group, 2,9-phenanthrolin-7-yl group, 2,9-phenanthrolin-8-yl group, 2,9-phenanthrolin-10-yl group, 2,8-phenanthrolin-1-yl group, 2,8-phenanthrolin-3-yl group, 2,8-phenanthrolin-4-yl group, 2,8-phenanthrolin-5-yl group, 2,8-phenanthrolin-6-yl group, 2,8-phenanthrolin-7-yl group, 2,8-phenanthrolin-9-yl group, 2,8-phenanthrolin-10-yl group, 2,7-phenanthrolin-1-yl group, 2,7-phenanthrolin-3-yl group, 2,7-phenanthrolin-4-yl group, 2,7-phenanthrolin-5-yl group, 2,7-phenanthrolin-6-yl group, 2,7-phenanthrolin-8-yl group, 2,7-phenanthrolin-9-yl group, 2,7-phenanthrolin-10-yl group, 1-phenazinyl group, 2-phenazinyl group, 1-phenothiadinyl group, 2-phenothiadinyl group, 3-phenothiadinyl group, 4-phenothiadinyl group, 1-phenoxadinyl group, 2-phenoxadinyl group, 3-phenoxadinyl group, 4-phenoxadinyl group, 2-oxazolyl group, 4-oxazolyl group, 5-oxazolyl group, 2-oxadiazolyl group, 5-oxadiazolyl group, 3-furazanyl group, 2-thienyl group, 3-thienyl group, 2-methylpyrrol-1-yl group, 2-methylpyrrol-3-yl group, 2-methylpyrrol-4-yl group, 2-methylpyrrol-5-yl group, 3-methylpyrrol-1-yl group, 3-methylpyrrol-2-yl group, 3-methylpyrrol-4-yl group, 3-methylpyrrol-5-yl group, 2-t-butyl-pyrrol-4-yl group, 3-(2-phenylpropyl)pyrrol-1-yl group, 2-methyl-1-indolyl group, 4-methyl-1-indolyl group, 2-methyl-3-indolyl group, 4-methyl-3-indolyl group, 2-t-butyl-1-indolyl group, 4-t-butyl-1-indolyl group, 2-t-butyl-3-indolyl group, and 4-t-butyl-3-indolyl group.

The substituted or unsubstituted carboxyl group having 1 to 50 carbon atoms is represented by -COOZ. Examples of Z include methyl, ethyl, propyl, isopropyl, n-butyl, s-butyl, isobutyl, t-butyl, n-pentyl, n-hexyl, n-heptyl, n-octyl, hydroxymethyl, 1-hydroxyethyl, 2-hydroxyethyl, 2-hydroxyisobutyl, 1,2-dihydroxyethyl, 1,3-dihydroxyisopropyl, 2,3-dihydroxy-t-butyl, 1,2,3-trihydroxypropyl, chloromethyl, 1-chloroethyl, 2-chloroethyl, 2-chloroisobutyl, 1,2-dichloroethyl, 1,3-dichloroisopropyl, 2,3-dichloro-t-butyl, 1,2,3-trichloropropyl, bromomethyl, 1-bromoethyl, 2-bromoethyl, 2-bromoisobutyl, 1,2-dibromoethyl, 1,3-dibromoisopropyl, 2,3-dibromo-t-butyl, 1,2,3-tribromopropyl, iodomethyl, 1-iodoethyl, 2-iodoethyl, 2-iodoisobutyl, 1,2-diiodoethyl, 1,3-diiodoisopropyl, 2,3-diiodo-t-butyl, 1,2,3-triiodopropyl, aminomethyl, 1-aminoethyl, 2-aminoethyl, 2-aminoisobutyl, 1,2-diaminoethyl, 1,3-diaminoisopropyl, 2,3-diamino-t-butyl, 1,2,3-triaminopropyl, cyanomethyl, 1-cyanoethyl, 2-cyanoethyl, 2-cyanoisobutyl, 1,2-dicyanoethyl, 1,3-dicyanoisopropyl, 2,3-dicyano-t-butyl, 1,2,3-tricyanopropyl, nitromethyl, 1-nitroethyl, 2-nitroethyl, 2-nitroisobutyl, 1,2-dinitroethyl, 1,3-dinitroisopropyl, 2,3-dinitro-t-butyl, and 1,2,3-trinitropropyl.

As examples of the substituted or unsubstituted styryl group, 2-phenyl-1-vinyl group, 2,2-diphenyl-1-vinyl group, 1,2,2-triphenyl-1-vinyl group, and the like can be given.

As examples of the halogen group, fluorine, chlorine, bromine, iodine, and the like can be given.

m is preferably 1 or 2, and n is preferably 0 to 4. When m≥2, the Ar's in (VI) may be the same or different. When n≥2, the X's in (VI) may be the same or different.

As the material used in the emitting layer, it is further preferable to use an anthracene derivative represented by the following formula (VII).

A¹-L-A² (VII)

wherein A¹ and A² are independently a substituted or unsubstituted monophenylanthryl group or substituted or unsubstitued diphenylanthryl group, and may be the same or different.

In addition to the anthracene derivative described above, an anthracene derivative represented by the formula (VIII) can be given.

A³-An-A⁴ (VIII)

wherein An is a substituted or unsubstituted divalent anthracene residue; and A³ and A⁴ are independently a substituted or unsubstituted monovalent condensed aromatic ring or a substituted or unsubstituted non-condensed ring aryl group having 12 or more carbon atoms and may be the same or different.

As the preferable anthracene derivative represented by the formula (VII), anthracene derivatives represented by the formula (VII-a) or the formula (VII-b) can be given, for example. 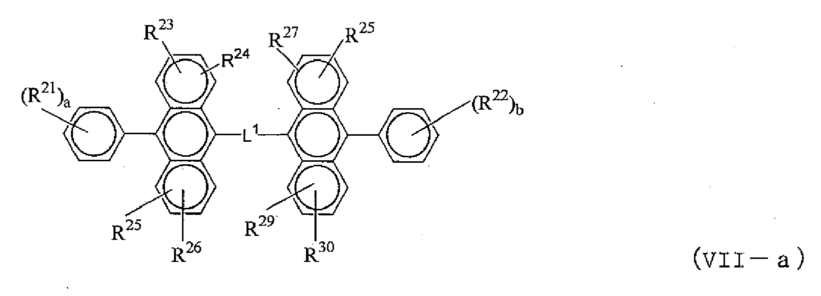 wherein R²¹ to R³⁰ are independently a hydrogen atom, an alkyl group, a cycloalkyl group, an aryl group which may be substituted, an alkoxy group, an aryloxy group, an alkylamino group, an arylamino group or a heterocyclic group which may be substituted; a and b are each an integer of 1 to 5; when they are 2 or more, R²¹s or R²²s may be the same or different, or R²¹s or R²²s may be bonded together to form a ring; R²³ and R²⁴, R²⁵ and R²⁶, R²⁷ and R²⁸, or R²⁹ and R³⁰ may be bonded together to form a ring; and L¹ is a single bond, -O-, -S-, -N(R)- (R is an alkyl group or a substituted or unsubstituted aryl group), or an arylene group.

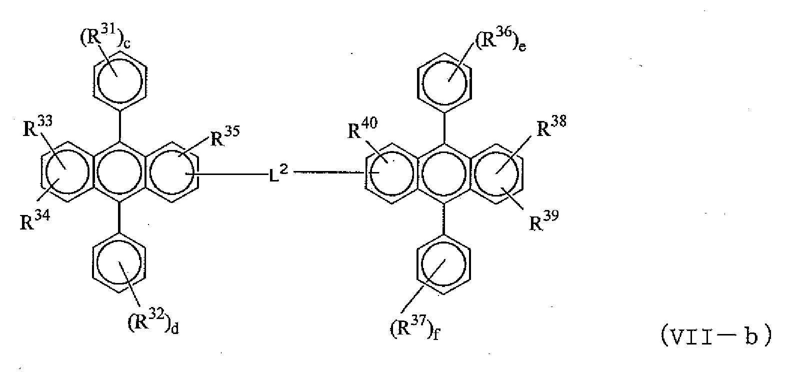

wherein R³¹ to R⁴⁰ are independently a hydrogen atom, an alkyl group, a cycloalkyl group, an aryl group which may be substituted, an alkoxy group, an aryloxy group, an alkylamino group, an arylamino group or a heterocyclic group which may be substituted; c, d, e and f are each an integer of 1 to 5; when they are 2 or more, R³¹s, R³²s_{,} R³⁶s or R³⁷s may be the same or different, R³¹s, R³²s, R³⁶s or R³⁷s may be bonded together to form a ring, or R³³ and R³⁴, or R³⁸ and R³⁹ may be bonded together to form a ring; and L² is a single bond, -O-, -S-, -N(R)- (R is an alkyl group or a substituted or unsubstituted aryl group), or an arylene group.
Here, the term "may be substituted" refers to "a substituted or unsubstituted".

As for R²¹ to R⁴⁰ shown in the above formulae (VII-a) and (VII-b), as the alkyl group, an alkyl group having 1 to 6 carbon atoms, as the cyclo group, a cyclo alkyl group having 3 to 6 carbon atoms, as the aryl group, an aryl group having 5 to 18 carbon atoms, as the alkoxy group, an alkoxy group having 1 to 6 carbon atoms, as the aryoxy group, an aryloxy group having 5 to 18 carbon atoms, as the arylamino group, an amino group substituted with an aryl group having 5 to 16 carbon atoms, as the heterocyclic group, triazole, oxadiazole, quinoxaline, furanyl, or thienyl or the like can preferably be given.

As the alkyl group and the aryl group represented by R in -N(R)- in L¹ and L², an alkyl group having 1 to 6 carbon atoms and an aryl group having 5 to 18 carbon atoms are preferable.

The emitting performance can be increased by further adding a slight amount of a fluorescent material as a dopant in the emitting layer. As such a dopant, a known emitting material having a long life may be used. It is preferable to use a material represented by the formula (IX) as the dopant of the emitting material. 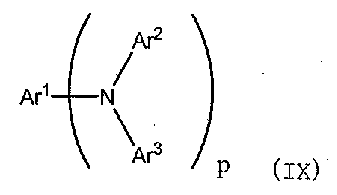 wherein Ar¹ to Ar³ are a substituted or unsubstituted aromatic group having 6 to 50 nucleus carbon atoms or a substituted or unsubstituted styryl group.

As examples of the substituted or unsubstituted aromatic group having 6 to 50 nucleus carbon atoms, a phenyl group, 1-naphthyl group, 2-naphthyl group, 1-anthryl group, 2-anthryl group, 9-anthryl group, 1-phenanthryl group, 2-phenanthryl group, 3-phenanthryl group, 4-phenanthryl group, phenanthryl group, 1-naphthacenyl group, 2-naphthacenyl group, 9-naphthacenyl group, and 1-pyrenyl group, 2-pyrenyl group, 4-pyrenyl group, 2-biphenylyl group, 3-biphenylyl group, 4-biphenylyl group, p-terphenyl-4-yl group, p-terphenyl-3-yl group, p-terphenyl-2-yl group, m-terphenyl-4-yl group, m-terphenyl-3-yl group, m-terphenyl-2-yl group, o-tolyl group, m-tolyl group, p-tolyl group, p-t-butylphenyl group, p-(2-phenylpropyl)phenyl group, 3-methyl-2-naphthyl group, 4-methyl-1-naphthyl group, 4-methyl-1-anthryl group, 4'-methylbiphenylyl group, 4"-t-butyl-p-terphenyl-4-yl group, 2-fluorenyl group, 9,9-dimethyl-2-fluorenyl group, 3-fluoranthenyl group, and the like can be given.

Of these, phenyl, 1-naphthyl, 2-naphthyl, 9-phenanthryl, 1-naphthacenyl, 2-naphthacenyl, 9-naphthacenyl, 1-pyrenyl, 2-pyrenyl, 4-pyrenyl, 2-biphenylyl, 3-biphenylyl, 4-biphenylyl, o-tolyl, m-tolyl, p-tolyl, p-t-butylphenyl, 2-fluorenyl, 9,9-dimethyl-2-fluorenyl, and 3-fluoranthenyl groups are preferable.
As examples of the substituted or unsubstituted styryl group, 2-phenyl-1-vinyl group, 2,2-diphenyl-1-vinyl group, 1,2,2-triphenyl-1-vinyl group, and the like can be given.

p is an integer of 1 to 4. When p≥2, the Ar² and Ar³ in (IX) may be the same or different.

### [Hole-injecting/transporting layer]

The hole-injecting/transporting layer is a layer for helping the injection of holes into the emitting layer to transport the holes to a light emitting region. The hole mobility thereof is large and the ionization energy thereof is usually as small as 5.5 eV or less. Such a hole-injecting/transporting layer is preferably made of a material which can transport holes to the emitting layer at a lower electric field intensity. The hole mobility thereof is preferably at least 10⁻⁴ cm²/V·second when an electric field of, e.g., 10⁴ to 10⁶ V/cm is applied.

As mentioned above, when the material for an organic EL device of the invention is used in the hole-transporting region, the hole-transporting layer may be formed using the compound of the invention singly or in combination with other materials. When the hole-transporting layer is formed using a mixture, it is preferable to mix a phenylenediamine compound represented by the above formula (V).
However, a compound to be mixed is not limited to the compound represented by the formula (V). A suitable compound may be appropriately selected from compounds generally used as the carrier-transporting material for the hole or known compounds used in the hole-injecting layer of an EL device.
When a region in addition to the hole-transporting region includes the material of the invention, the hole-transporting layer may be formed using the following mixed materials singly.

Specific examples of mixed materials include triazole derivatives (see USP No. 3,112,197 or the like), oxadiazole derivatives (see USP No. 3,189,447 or the like), imidazole derivatives (see JP-B-37-16096 or the like), polyarylalkane derivatives (see USP Nos. 3,615,402, 3,820,989 and 3,542,544, JP-B-45-555 and 51-10983, JP-A-51-93224, 55-17105, 56-4148, 55-108667, 55-156953 and 56-36656, or the like), pyrazoline derivatives and pyrazolone derivatives (see USP Nos. 3,180,729 and 4,278,746, JP-A-55-88064, 55-88065, 49-105537, 55-51086, 56-80051, 56-88141, 57-45545, 54-112637 and 55-74546, or the like), phenylenediamine derivatives (see USP No. 3,615,404, JP-B-51-10105, 46-3712 and 47-25336, JP-A-54-53435, 54-110536 and 54-119925, or the like), arylamine derivatives (see USP Nos. 3,567,450, 3,180,703, 3,240,597, 3,658,520, 4,232,103, 4,175,961 and 4,012,376, JP-B-49-35702 and 39-27577, JP-A-55-144250, 56-119132 and 56-22437, DE1,110,518, or the like), amino-substituted chalcone derivatives (see USP No. 3,526,501, or the like), oxazole derivatives (ones disclosed in USP No. 3,257,203, or the like), styrylanthracene derivatives (see JP-A-56-46234, or the like), fluorenone derivatives (JP-A-54-110837, or the like), hydrazone derivatives (see USP Nos. 3,717,462, JP-A-54-59143, 55-52063, 55-52064, 55-46760, 55-85495, 57-11350, 57-148749 and 2-311591, or the like), stilbene derivatives (see JP-A-61-210363, 61-228451, 61-14642, 61-72255, 62-47646, 62-36674, 62-10652, 62-30255, 60-93455, 60-94462, 60-174749 and 60-175052, or the like), silazane derivatives (USP No. 4,950,950), polysilanes (JP-A-2-204996), aniline copolymers (JP-A-2-282263), and electroconductive high molecular oligomers (in particular thiophene oligomers) disclosed in JP-A-1-211399.

In addition to the hole-transporting layer, in order to help the injection of holes, it is preferred that the hole-injecting layer be provided separately. As the material for the hole-injecting layer, the material of the organic EL of the invention may be used singly or in combination with other materials. As the other materials, the same materials used for the hole-transporting layer can be used. The following can also be used, in addition to the compound shown in the above formula (V): porphyrin compounds (disclosed in JP-A-63-2956965 or the like), aromatic tertiary amine compounds, and styrylamine compounds (see USP No. 4,127,412, JP-A-53-27033, 54-58445, 54-149634, 54-64299, 55-79450, 55-144250, 56-119132, 61-295558, 61-98353 and 63-295695, or the like). Of these, the aromatic tertiary amine compounds are particularly preferable.

The following can also be given as examples: 4,4'-bis(N-(1-naphthyl)-N-phenylamino)biphenyl, which has in the molecule thereof two condensed aromatic rings, disclosed in USP No. 5,061,569, 4,4',4"-tris(N-(3-methylphenyl)-N-phenylamino)triphenylamine, wherein three triphenylamine units (hereinafter referred to as "MTDATA") are linked to each other in a star-burst form, disclosed in JP-A-4-308688, and the like.

In addition to the aromatic dimethylidene type compounds, inorganic compounds such as p-type Si and p-type SiC can also be used as the material for the hole-injecting layer or the hole-transporting layer.

The hole-injecting/transporting layer can be formed by making the above-mentioned materials into a thin film by a known method, such as vacuum deposition, spin coating, casting or LB technique. The film thickness of the hole-injecting/transporting layer is not particularly limited, and is usually from 5 nm to 5 µm. This hole-injecting layer or the hole-transporting layer may be a single layer made of one or more of the above-mentioned materials, or may be stacked hole-injecting layers or hole-transporting layers made of different compounds, insofar as the compound of the invention is contained.

The organic semiconductor layer, which is a part of the hole-transporting layer, is a layer for helping the injection of holes or electrons into the emitting layer, and is preferably a layer having an electric conductivity of 10⁻¹⁰ S/cm or more. As the material for such an organic semiconductor layer, electroconductive oligomers such as thiophene-containing oligomers or arylamine-containing oligomers disclosed in JP-A-8-193191, and electroconductive dendrimers such as arylamine-containing dendrimers may be used.

### [Electron-injecting/transporting layer]

An electron-injecting layer (often described as "an electron-transporting layer"), is a layer which assists injection of electrons into the emission layer, and exhibits a high electron mobility. An adhesion-improving layer is formed of a material which exhibits excellent adhesion to the cathode. As the material used for the electron injecting/transporting layer, 8-hydroxyquinoline, a metal complex of an 8-hydroxyquinoline derivative, and a compound having a nitrogen-containing heterocyclic ring are suitable.

As specific examples of 8-hydroxyquinoline and a metal complex of an 8-hydroxyquinoline derivative, metal chelate oxinoid compounds including a chelate of oxine (8-quinolinol or 8-hydroxyquinoline) can be given.
For example, Alq described referring to the emitting material may be used for the electron-injecting layer.

An electron-transporting compound of the following formula can be given as the oxadiazole derivative. 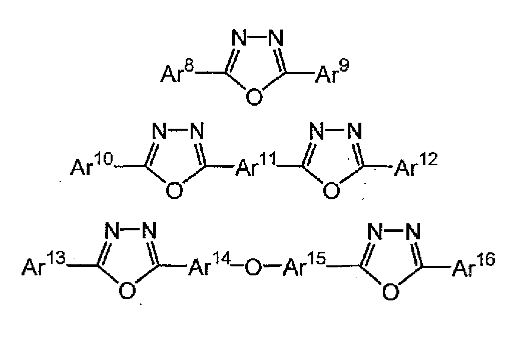 wherein Ar⁸, Ar⁹, Ar¹⁰, Ar¹², Ar¹³, and Ar¹⁶ are independently substituted or unsubstituted aryl groups and may be the same or different. Ar¹¹, Ar¹⁴, and Ar¹⁵ are independently substituted or unsubstituted arylene groups and may be the same or different.

As examples of the aryl group, a phenyl group, a biphenyl group, an anthranyl group, a perylenyl group, and a pyrenyl group can be given. As examples of the arylene group, a phenylene group, a naphthylene group, a biphenylene group, an anthranylene group, a perylenylene group, a pyrenylene group, and the like can be given. As the substituent, an alkyl group having 1 to 10 carbon atoms, an alkoxy group having 1 to 10 carbon atoms, a cyano group, and the like can be given. The electron transporting compound is preferably one from which a thin film can be formed.

The following compounds can be given as specific examples of the electron transporting compound.

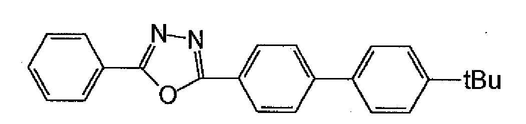

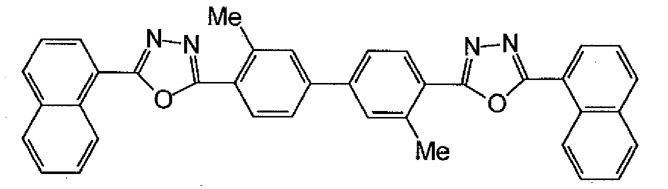

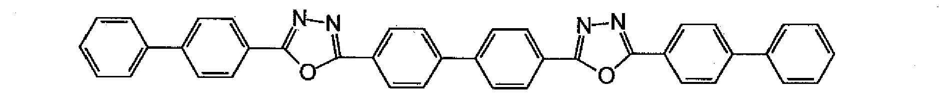

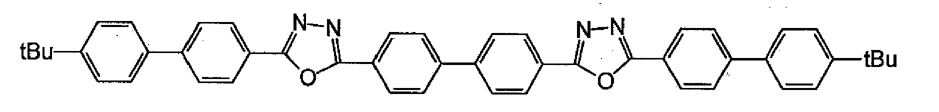

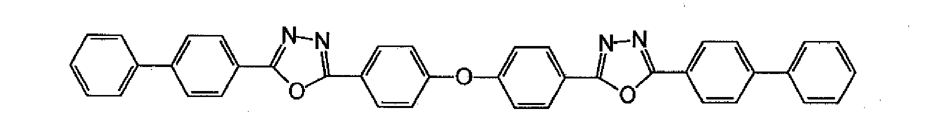

A preferred embodiment of the invention is a device containing a reducing dopant in an interfacial region between its electron transferring region or cathode and organic layer. The reducing dopant is defined as a substance which can reduce an electron-transferring compound. Accordingly, various substances which have given reducing properties can be used. For example, at least one substance can be preferably used which is selected from the group consisting of alkali metals, alkaline earth metals, rare earth metals, alkali metal oxides, alkali metal halides, alkaline earth metal oxides, alkaline earth metal halides, rare earth metal oxides, rare earth metal halides, alkali metal organic complexes, alkaline earth metal organic complexes, and rare earth metal organic complexes.

More specific examples of the preferred reducing dopants include at least one alkali metal selected from the group consisting of Na (work function: 2.36 eV), K (work function: 2.28 eV), Rb (work function: 2.16 eV) and Cs (work function: 1.95 eV), and at least one alkaline earth metal selected from the group consisting of Ca (work function: 2.9 eV), Sr (work function: 2.0 to 2.5 eV), and Ba (work function: 2.52 eV). Metals having a work function of 2.9 eV or less are in particular preferred.
Of these, a more preferable reducing dopant is at least one alkali metal selected from the group consisting of K, Rb and Cs. Even more preferable is Rb or Cs. Most preferable is Cs.

These alkali metals are particularly high in reducing ability. Thus, the addition of a relatively small amount thereof to an electron injecting zone makes it possible to improve the luminance of the organic EL device and make the lifetime thereof long. As the reducing dopant having a work function of 2.9 eV or less, any combination of two or more out of these alkali metals is also preferred. Particularly preferred is any combination containing Cs, for example, combinations of Cs and Na, Cs and K, Cs and Rb, or Cs, Na and K.
The combination containing Cs makes it possible to exhibit the reducing ability efficiently. The luminance of the organic EL device can be improved and the lifetime thereof can be made long by the addition thereof to its electron-injecting zone.

In the invention, an electron-injecting layer made of an insulator or a semiconductor may further be provided between a cathode and an organic layer. By providing the layer, current leakage can be effectively prevented to improve the injection of electrons.
As the insulator, at least one metal compound selected from the group consisting of alkali metal calcogenides, alkaline earth metal calcogenides, halides of alkali metals and halides of alkaline earth metals can be preferably used. When the electron-injecting layer is formed of the alkali metal calcogenide or the like, the injection of electrons can be preferably further improved.

Specifically preferable alkali metal calcogenides include Li₂O, LiO, Na₂S, Na₂Se and NaO and preferable alkaline earth metal calcogenides include CaO, BaO, SrO, BeO, BaS and CaSe. Preferable halides of alkali metals include LiF, NaF, KF, Licl, KCl, and NaCl. Preferable halides of alkaline earth metals include fluorides such as CaF₂, BaF₂, SrF₂, and MgF₂ and BeF₂ and halides in addition to fluorides.

Examples of the semiconductor for forming an electron-injecting layer include oxides, nitrides or oxynitrides containing at least one element selected from Ba, Ca, Sr, Yb, A1, Ga, In, Li, Na, Cd, Mg, Si, Ta, Sb and Zn, and combinations of two or more thereof. The inorganic compound for forming an electron-injecting layer is preferably a microcrystalline or amorphous insulating thin film. When an electron-injecting layer is formed of the insulating thin film, a more uniform thin film can be formed to reduce pixel defects such as dark spots.
Examples of such an inorganic compound include the above-mentioned alkali metal calcogenides, alkaline earth metal calcogenides, halides of alkali metals, and halides of alkaline earth metals.

### [Cathode]

For the cathode, the following may be used: an electrode substance made of a metal, an alloy or an electroconductive compound, or a mixture thereof which has a small work function (4 eV or less). Specific examples of the electrode substance include sodium, sodium-potassium alloy, magnesium, lithium, magnesium/silver alloy, aluminum/aluminum oxide, aluminum/lithium alloy, indium, and rare earth metals.

This cathode can be formed by making the electrode substances into a thin film by vapor deposition, sputtering or some other method.

In the case where emission from the emitting layer is outcoupled through the cathode, it is preferred to make the transmittance of the cathode to the emission larger than 10%.
The sheet resistance of the cathode is preferably several hundreds Ω/□ or less, and the film thickness thereof is usually from 10 nm to 1 µm, preferably from 50 to 200 nm.

### [Insulative layer]

In the organic EL device, pixel defects based on leakage or a short circuit are easily generated since an electric field is applied to the super thin film. In order to prevent this, it is preferred to insert an insulator thin layer between the pair of electrodes.
Examples of the material used in the insulative layer include aluminum oxide, lithium fluoride, lithium oxide, cesium fluoride, cesium oxide, magnesium oxide, magnesium fluoride, calcium oxide, calcium fluoride, cesium fluoride, cesium carbonate, aluminum nitride, titanium oxide, silicon oxide, germanium oxide, silicon nitride, boron nitride, molybdenum oxide, ruthenium oxide, and vanadium oxide.
A mixture or laminate thereof may be used.

### [Example of fabricating organic EL device]

The organic EL device can be fabricated by forming an anode and an emitting layer, optionally forming a hole-injecting layer and an electron-injecting layer if necessary, and further forming a cathode by use of the materials and methods exemplified above. The organic EL device can be fabricated in the order reverse to the above, i.e., the order from a cathode to an anode.

An example of the fabrication of the organic EL device will be described below which has a structure wherein the following are successively formed on a transparent substrate: anode/hole-transporting layer/emitting layer/electron-transporting layer/cathode.
First, a thin film made of an anode material is formed into a thickness of 1 µm or less, preferably 10 to 200 nm on an appropriate transparent substrate by vapor deposition, sputtering or some other method, thereby forming an anode.
Next, a hole-transporting layer is formed on this anode. As described above, the hole-transporting layer can be formed by vacuum deposition, spin coating, casting, LB technique, or some other method. Vacuum deposition is preferred since a homogenous film is easily obtained and pinholes are not easily generated.

In the case where the hole-transporting layer is formed by vacuum deposition, conditions for the deposition vary depending upon the compound used (materials of the hole-transporting layer), the desired crystal structure or recombining structure of the hole-transporting layer, or the like. In general, the conditions are preferably selected from the following: deposition source temperature of 50 to 450°C, vacuum degree of 10⁻⁷ to 10⁻³ torr, vapor deposition rate of 0.01 to 50 nm/second, substrate temperature of -50 to 300°C, and film thickness of 5 nm to 5 µm.

Next, an emitting layer is disposed on the hole-transporting layer. The emitting layer can also be formed by using a desired organic luminescent material and making the material into a thin film by vacuum deposition, sputtering, spin coating, casting or some other method. Vacuum deposition is preferred since a homogenous film is easily obtained and pinholes are not easily generated. In the case where the emitting layer is formed by vacuum deposition, conditions for the deposition, which vary depending on the compound used, can be generally selected from conditions similar to those for the hole-transporting layer.

Next, an electron-transporting layer is formed on this emitting layer. Like the hole-transporting layer and the emitting layer, the layer is preferably formed by vacuum deposition because a homogenous film is required. Conditions for the deposition can be selected from conditions similar to those for the hole-transporting layer and the emitting layer.

Lastly, a cathode is stacked thereon to obtain an organic EL device.
The cathode is made of a metal, and vapor deposition or sputtering may be used. However, vacuum deposition is preferred in order to protect underlying organic layers from being damaged when the cathode film is formed.
For the organic EL device fabrication that has been described above, it is preferred that the formation from the anode to the cathode is continuously carried out, using only one vacuuming operation.

A method for forming each of the layers constituting the organic EL device of the invention is not particularly limited. A known forming method, such as vacuum deposition, spin coating or the like can be used. The organic thin layer containing the material for the organic EL device of the invention can be formed by a known method, such as vacuum deposition, molecular beam deposition (MBE method), or coating method such as dipping, spin coating, casting, casting, bar coating and roll coating using a solution obtained by dissolving materials in a solvent.

The film thickness of each of the organic layers in the organic EL device of the invention is not particularly limited. In general, defects such as pinholes are easily generated when the film thickness is too small. Conversely, a high applied voltage becomes necessary, leading to low efficiency, when the film thickness is too large. Usually, therefore, the film thickness is preferably in the range of several nanometers to one micrometer.

The organic EL device emits light when applying a voltage between electrodes. If a DC voltage is applied to the organic EL device, emission can be observed when the polarities of the anode and the cathode are positive and negative, respectively, and a DC voltage of 5 to 40 V is applied. When a voltage with an opposite polarity is applied, no electric current flows and hence, emission does not occur. If an AC voltage is applied, uniform emission can be observed only when the cathode and the anode have a positive polarity and a negative polarity, respectively. The waveform of the AC applied may be arbitrary.

### EXAMPLES

The material for an organic EL device and the organic EL device of the invention will be described in detail referring to the following examples, which should not be construed as limiting the scope of the invention.
The structures of the compounds synthesized or used in the examples are shown below.

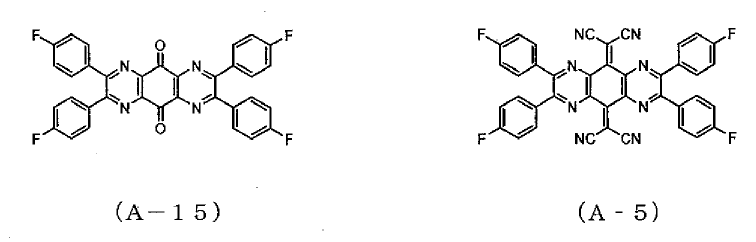

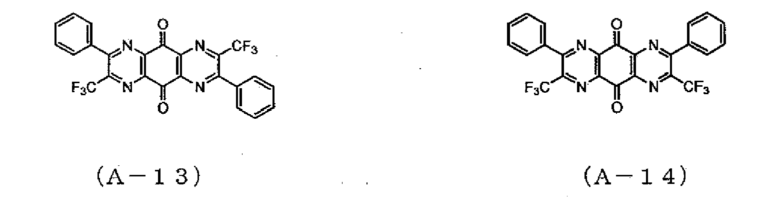

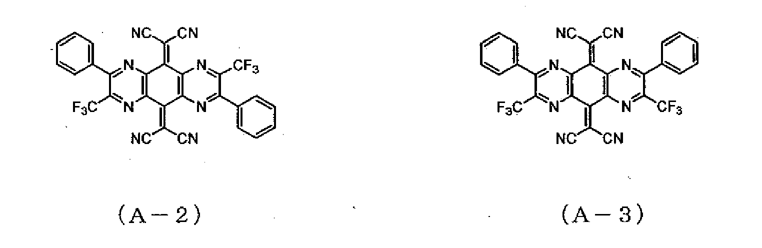

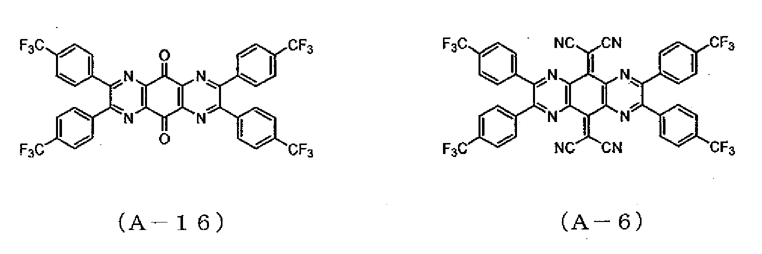

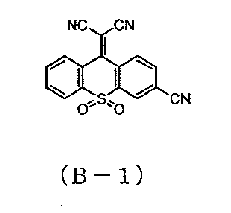

### [Material for organic EL device]

### Example 1

### Synthesis of a compound represented by the formula (A-15)

1.6 g of tetraminodiphenoquinone synthesized according to the method described in Justus Liebigs Ann. Chem., 667, p. 55-71 (1963) and 5.0 g of 4,4'-difluorobenzyl were added to 50 ml of acetic acid. The resulting mixture was heated at 80°C for three hours with stirring. After cooling, the reaction liquid was condensed. A precipitated solid product was filtered, and recrystallized from acetonitrile, whereby 2.7 g of an orange red solid product represented by the formula (A-15) was obtained.
As a result of an IR measurement of the compound, absorption of a carbonyl group was observed at 1705 cm⁻¹. Mass spectrometry revealed that the compound had a peak at an M/Z of 588.
The compound was then dissolved in acetonitrile so that the concentration became 0.01 mol/l. A reduction potential was measured by cyclic voltammetry using tetrabutylammonium perchlorate (TBAP) as a supporting electrode and a saturated calomel electrode (SCE) as a reference electrode. The reduction potential was found to be -0.4 V.

### Example 2

### Synthesis of a compound represented by the formula (A-5)

2.0 g of the compound represented by the formula (A-15) synthesized in Example 1 and 0.6 g of malononitrile were mixed with 70 ml of methylene chloride. To the resulting mixture, 2.5 g of titanium tetrachloride was dripped in a nitrogen atmosphere for 20 minutes while cooling the mixture on ice. Subsequently, 20 ml of pyridine was dripped for 20 minutes. After stirring for five hours at room temperature, 50 ml of 10% aqueous hydrochloric acid was added. The methylene chloride was distilled off under reduced pressure, and a precipitate was filtered off and dried. The precipitate was recrystallized from acetonitrile, followed by sublimation and purification, whereby 1.2 g of a compound represented by the formula (A-5) was obtained.
As a result of an IR measurement of the compound, absorption of a cyano group was observed at 2218 cm⁻¹ and disappearance of the absorption of the carbonyl group at 1705 cm⁻¹ was confirmed. Mass spectrometry revealed that the compound had a peak at an M/Z of 684.
The reduction potential was measured by cyclic voltammetry in the same manner as in Example 1, and was found to be 0.13 V.

### Example 3

### Synthesis of a mixture of a compound represented by the formula (A-13) and a compound represented by the formula (A-14)

The same procedures as in Example 1 were followed, except that 5.0 g of 3,3,3,-trifluoro-1-phenyl-1,2-propanedione monohydrate was used instead of 5.0 g of 4,4'-difluorobenzyl, whereby 1.9 g of a mixture of a compound represented by the formula (A-13) and a compound represented by the formula (A-14) was obtained.
As a result of an IR measurement of the mixture, absorption of a carbonyl group was confirmed at 1706 cm⁻¹. Mass spectrometry revealed that the compound had a peak at an M/Z of 500.
The reduction potential of the mixture was measured by cyclic voltammetry in the same manner as in Example 1, and was found to be -0.2 V.

### Example 4

### Synthesis of a mixture of a compound represented by the formula (A-2) and a compound represented by the formula (A-3)

The same procedures as in Example 2 were followed, except that 1.9 g of the mixture of the compound represented by the formula (A-13) and the compound represented by the formula (A-14) synthesized in Example 3 was used instead of the compound represented by the formula (A-15) of Example 2, whereby 1.1 g of a mixture of a compound represented by the formula (A-2) and a compound represented by the formula (A-3) was obtained.
As a result of an IR measurement of the mixture, absorption of a cyano group was observed at 2220 cm⁻¹ and disappearance of the absorption of a carbonyl group at 1706 cm⁻¹ was confirmed. Mass spectrometry revealed that the compound had a peak at an M/Z of 596.
The reduction potential of the mixture was measured by cyclic voltammetry in the same manner as in Example 1, and was found to be 0.41 V.

### Example 5

### Synthesis of a compound represented by the formula (A-16)

The same procedures as in Example 1 were followed, except that 7.0 g of 4,4'-bis(trifluoromethyl)benzyl was used instead of 5.0 g of 4,4'-difluorobenzyl of Example 1, whereby 2.8 g of a compound represented by the formula (A-16) was obtained.
As a result of an IR measurement of the compound, absorption of a carbonyl group was observed at 1705 cm⁻¹. Mass spectrometry revealed that the compound had a peak at an M/Z of 788.
The reduction potential of the compound was measured by cyclic voltammetry in the same manner as in Example 1, and was found to be -0.24 V.

### Example 6

### Synthesis of a compound represented by the formula (A-6)

The same procedures as in Example 2 were followed, except that 2.5 g of the compound represented by the formula (A-16) synthesized in Example 5 was used instead of the compound represented by the formula (A-153, whereby 1.9 g of a compound represented by the formula (A-6) was obtained.
As a result of an IR measurement of the compound, absorption of a cyano group was observed at 2218 cm⁻¹ and disappearance of the absorption of a carbonyl group at 1705 cm⁻¹ was confirmed. Mass spectrometry revealed that the compound had a peak at an M/Z of 836.
The reduction potential of the compound was measured by cyclic voltammetry in the same manner as in Example 1, and was found to be 0.26 V.

### Example 7

### Synthesis of a compound represented by the formula (B-1)

2.7 g of 9-oxo-9H-thioxthantene-3-carbonitrile and 0.82 g of malononitrile were added to 150 ml of methylene chloride. To the resulting mixture, 2.8 g of titanium tetrachloride was dripped under a nitrogen atmosphere for 25 minutes while cooling the mixture on ice. Subsequently, 10 ml of pyridine was dripped for 30 minutes. After stirring for five hours at room temperature, 50 ml of 10% aqueous hydrochloric acid was added. The methylene chloride was distilled off under reduced pressure. A precipitate was filtered off, washed with water and then methanol, and dried. The precipitate was then recrystallized from acetonitrile, followed by sublimation and purification, whereby 2.5 g of a white crystal was obtained.
As a result of an IR measurement of the compound, absorption of a cyano group was observed at 2240 cm⁻¹ . Mass spectrometry revealed that the compound had a peak at an M/Z of 317.
The reduction potential of the compound was measured by cyclic voltammetry in the same manner as in Example 1, and was found to be -0.36 V.

### [Organic EL device]

### Example 8

A 25 mm x 75 mm glass substrate with a thickness of 1.1 mm provided with transparent electrodes formed of ITO (manufactured by Geomatics Corporation) was subjected to ultrasonic cleaning for five minutes in isopropyl alcohol, followed by UV/ozone cleaning for 30 minutes.
The cleaned glass substrate having the transparent electrode lines was then secured to a substrate holder of an apparatus for vacuum deposition. First, the compound represented by the formula (A-5) synthesized in Example 2 and the compound represented by the following formula (C-1) were deposited onto the surface of the glass substrate on which the transparent electrode lines are formed so as to cover the transparent electrodes, thereby forming a 60 nm-thick film in which the compound of the formula (A-5) and the compound of the formula (C-1) were mixed at a molar ratio of 2:98. The film of the compound mixture served as a hole-injecting layer.
Subsequently, a 20 nm-thick film of a compound represented by the following formula (HTM-1) was formed on the above-obtained film of the compound mixture. The film served as a hole-transporting layer.
Further, EM1 with a thickness of 40 nm was deposited thereon to form a film. Simultaneously, an amine compound D1 having the following styryl-group as an emitting molecule was deposited such that the weight ratio of EM1 and D1 was 40:2. The film served as an emitting layer.
A 10 nm-thick Alq film was formed on the above-obtained film. The film serves as an electron-injecting layer. Then, Li as a reductive dopant (Li source: manufactured by SAES Getters Co., Ltd.) and Alq were codeposited, whereby an Alq:Li film (film thickness: 10 nm) was formed as an electron-injecting layer (cathode). Metal aluminum was deposited on the Alq:Li film to form a metallic cathode, whereby an organic EL emitting device was fabricated.

The organic EL device was evaluated by measuring a driving voltage at a current density of 10 mA/cm² and a half life of luminance at an initial luminance of 1,000 nits, at room temperature, and with a DC constant power supply. The results obtained are shown in Table 1.

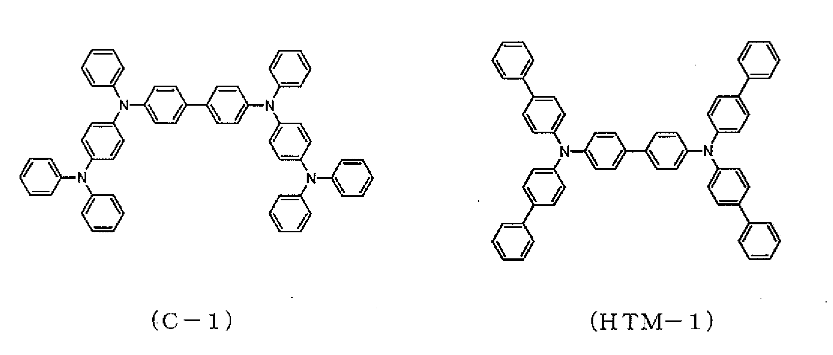

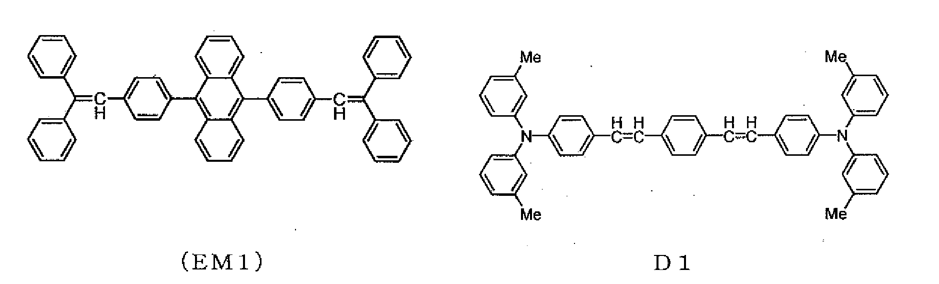

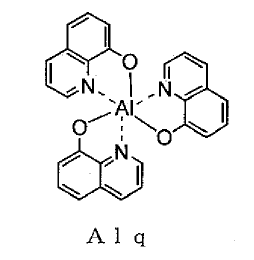

### Comparative Example 1

An organic EL device was fabricated and evaluated in the same manner as in Example 8, except that the hole-injecting layer was formed using the compound represented by the formula (C-1) singly.
The results obtained are shown in Table 1.

**[Table 1]**

| | Constitution materials for hole-injecting layer | Driving voltage(V) | Half life (hr) |
|---|---|---|---|
| Example 8 | Compounds of formulae (A-5) and (C-1) | 5.9 | 6,900 |
| Comparative Example 1 | Compound of formula (C-1) | 6.6 | 5,000 |

### INDUSTRIAL APPLICABILITY

The material for an organic EL device of the invention is suitable as a constitution material of an organic EL device, in particular, a hole-transporting layer or a hole-injecting layer. The material for an organic EL device of the invention can also be used as a charge-transporting material of an electrophotographic photoreceptor.
The organic EL device of the invention can be suitably used as a light source such as a planar emitting material and backlight of a display, a display part of a portable phone, PDA, a car navigator, or an instruction panel of an automobile, an illuminator, and the like.

## Claims

1. A material for an organic electroluminescent device represented by the following formula (I): 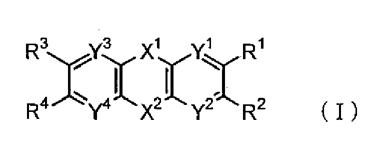 wherein X¹ and X² are independently one of the following divalent groups; Y¹ to Y⁴ are independently a carbon atom or a nitrogen atom; R¹ to R⁴ are independently a hydrogen atom, an alkyl group, an aryl group, a heterocyclic group, a halogen atom, a fluoroalkyl group, or a cyano group; and R¹ and R² , and R³ and R⁴ may be bonded together to form a ring, respectively.

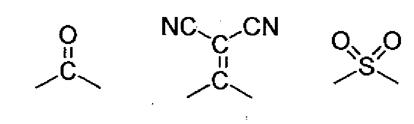

2. The material for an organic electroluminescent device according to claim 1, which is a compound represented by the following formula (II) or (III): 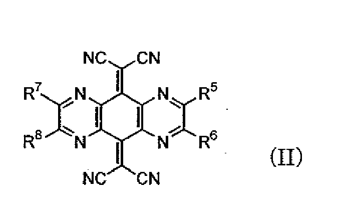 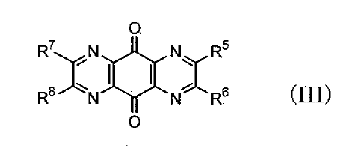 wherein R⁵ to R⁸ are independently a hydrogen atom, an alkyl group, an aryl group, a heterocyclic group, a halogen atom, a fluoroalkyl group or a cyano group; and R⁵ and R⁶ and R⁷ and R⁸ may be bonded together to form a ring, respectively.

3. The material for an organic electroluminescent device according to claim 1, which is a compound represented by the following formula (IV): 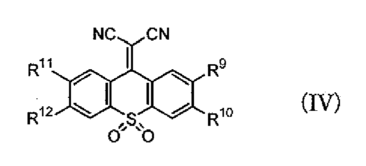 wherein R⁹ to R¹² are independently a hydrogen atom, an aryl group, a heterocyclic group, a halogen atom, a fluoroalkyl group, or a cyano group.

4. The material for an organic electroluminescent device according to any one of claims 1 to 3, wherein an acetonitrile solution has a reduction potential of -0.5 V (vsSCE) or more.

5. An organic electroluminescent device comprising a cathode and an anode, and one or a plurality of organic thin layers including an emitting layer being interposed between the cathode and the anode;
at least one of the organic thin layers containing the material for an organic electroluminescent device according to any one of claims 1 to 4.

6. The organic electroluminescent device according to claim 5, wherein the organic thin layers are a multilayer body in which a hole-transporting layer, an emitting layer, and an electron-transporting layer are stacked in this order from the anode.

7. The organic electroluminescent device according to claim 6, wherein the hole-transporting layer contains the material for an organic electroluminescent device.

8. The organic electroluminescent device according to claim 5, wherein the organic thin layers are a multilayer body in which a hole-injection layer, a hole-transporting layer, an emitting layer, and an electron-transporting layer are stacked in this order from the anode, and the hole-injection layer contains the material for an organic electroluminescent device.

9. The organic electroluminescent device according to claim 7 or 8, wherein the hole-transporting layer containing the material for an organic electroluminescent device further contains a phenylenediamine compound represented by the following formula (V): 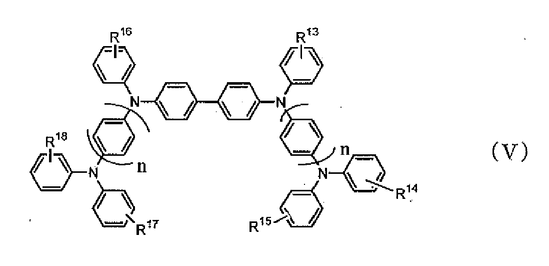 wherein R¹³ to R¹⁸ are independently a hydrogen atom, a halogen atom, a trifluoromethyl group, an alkyl group, an aryl group, or a heterocyclic group or R¹³ to R¹⁸ may form a naphthalene skeleton, a carbazole skeleton, or a fluorene skeleton with a phenyl group bonded; and n represents 1 or 2.
